# FASCICULE DE BREVET EUROPEEN

(11) **EP 1 919 513 B1**
(45) Date de publication et mention de la délivrance du brevet: **08.06.2011**
(21) Numéro de dépôt: 06778874.5
(22) Date de dépôt: 18.07.2006
(51) Int. Cl.: A61K 49/00, A61K 38/04, A61K 38/08, A61K 38/12, A61P 35/00, C07K 19/00, C07K 7/64, A61B 5/00

(54) **Vecteur ciblé comprenant le cyclodecapeptide RAFT et une fonction d' imagerie activable**
Zielgerichteter Vektor mit RAFT-cyclodecapeptid und einer aktivierbarer Bildgebungs-Funktion
Targeted vector comprising a cyclodecapeptide RAFT and an activable imaging function

(30) Priorité: 21.07.2005 FR 0507784
(43) Date de publication de la demande: 14.05.2008
(73) Titulaire: Commissariat à l'Énergie Atomique et aux Énergies Alternatives, 75015 Paris (FR); INSTITUT NATIONAL DE LA SANTE ET DE LA RECHERCHE MEDICALE (INSERM), 75013 Paris (FR); Université Joseph Fourier, 38041 Grenoble Cédex 9 (FR)
(72) Inventeur: TEXIER-NOGUES, Isabelle, F-38000 Grenoble (FR); COLL, Jean-Luc, F-38640 Claix (FR); DUMY, Pascal, F-38580 Allevard (FR); BOTURYN, Didier, F-38500 La Buisse (FR); FAVROT, Marie, F-38700 Corenc (FR)
(74) Mandataire: Marcadé, Véronique
(86) Numéro de dépôt international: PCT/FR2006/001749
(87) Numéro de publication internationale: WO 2007/010128

(56) Documents cités:
- WO-A-02/00265
- WO-A-2004/026894
- US-A1- 2005 142 068
- JOSEPHSON LEE ET AL: "Near-infrared fluorescent nanoparticles as combined MR/optical imaging probes" BIOCONJUGATE CHEMISTRY, ACS, WASHINGTON, DC, US, vol. 13, no. 3, mai 2002 (2002-05), pages 554-560, XP002267675 ISSN: 1043-1802
- CURNIS FLAVIO ET AL: "Differential binding of drugs containing the NGR motif to CD13 isoforms in tumor vessels, epithelia, and myeloid cells" 1 février 2002 (2002-02-01), CANCER RESEARCH, AMERICAN ASSOCIATION FOR CANCER RESEARCH, BALTIMORE, MD, US, PAGE(S) 867-874 , XP002215805 ISSN: 0008-5472 page 867, colonne 1 - page 872, colonne 1
- DATABASE BIOSIS [Online] BIOSCIENCES INFORMATION SERVICE, PHILADELPHIA, PA, US; 1 mars 2002 (2002-03-01), VAN HENSBERGEN YVETTE ET AL: "A doxorabicin-CNGRC-peptide conjugate with prodrug properties" XP002305933 Database accession no. PREV200200296740 & BIOCHEMICAL PHARMACOLOGY, vol. 63, no. 5, 1 mars 2002 (2002-03-01), pages 897-908, ISSN: 0006-2952
- BOTURYN DIDIER ET AL: "Template assembled cyclopeptides as multimeric system for integrin targeting and endocytosis" JOURNAL OF THE AMERICAN CHEMICAL SOCIETY, vol. 126, no. 18, 12 mai 2004 (2004-05-12), pages 5730-5739, XP002377072 ISSN: 0002-7863 cité dans la demande
- GARANGER ET AL: "New Multifunctional Molecular Conjugate Vector for Targeting, Imaging, and Therapy of Tumors" décembre 2005 (2005-12), MOLECULAR THERAPY, ACADEMIC PRESS, SAN DIEGO, CA,, US, PAGE(S) 1168-1175 , XP005176625 ISSN: 1525-0016 le document en entier
- LAW B ET AL: "DESIGN, SYNTHESIS AND CHARACTERIZATION OF UROKINASE PLASMINOGEN-ACTIVATOR-SENSITIVE NEAR-INFRARED REPORTER" CHEMISTRY AND BIOLOGY, CURRENT BIOLOGY, LONDON, GB, vol. 11, no. 1, janvier 2004 (2004-01), pages 99-106, XP008058421 ISSN: 1074-5521
- CHEN X ET AL: "In vivo Near-Infrared Fluorescence Imaging of Integrin .alpha.v.beta.3 in Brain Tumor Xenografts" CANCER RESEARCH, AMERICAN ASSOCIATION FOR CANCER RESEARCH, BALTIMORE, MD, US, vol. 64, no. 21, 1 novembre 2004 (2004-11-01), pages 8009-8014, XP002346959 ISSN: 0008-5472
- ARAP W ET AL: "CANCER TREATMENT BY TARGETED DRUG DELIVERY TO TUMOR VASCULATURE IN A MOUSE MODEL" SCIENCE, AMERICAN ASSOCIATION FOR THE ADVANCEMENT OF SCIENCE,, US, vol. 279, 16 janvier 1998 (1998-01-16), pages 377-380, XP000857470 ISSN: 0036-8075
- BRITZ-CUNNINGHAM SCOTT H. ET AL: 'Molecular targeting with radionuclides: State of the science.' JOURNAL OF NUCLEAR MEDICINE vol. 44, no. 12, Décembre 2003, pages 1945 - 1961 ISSN: 0161-5505
- WICKHAM T.J.: 'Ligand-directed targeting of genes to the site of disease' NATURE MEDICINE, NATURE PUBLISHING GROUP, NEW YORK, NY, US LNKD- DOI:10.1038/NM0103-135 vol. 9, no. 1, 01 Janvier 2003, pages 135 - 139, XP002282877 ISSN: 1078-8956
- BYRNE J.D. ET AL: 'Active targeting schemes for nanoparticle systems in cancer therapeutics' ADVANCED DRUG DELIVERY REVIEWS, ELSEVIER BV, AMSTERDAM, NL LNKD- DOI:10.1016/J.ADDR.2008.08.005 vol. 60, no. 15, 14 Décembre 2008, pages 1615 - 1626, XP025656956 ISSN: 0169-409X
- FOILLARD STÉPHANIE ET AL: "Targeted delivery of activatable fluorescent pro-apoptotic peptide into live cells." ORGANIC & BIOMOLECULAR CHEMISTRY 21 JAN 2009 LNKD- PUBMED:19109663, vol. 7, no. 2, 21 January 2009 (2009-01-21), pages 221-224, ISSN: 1477-0539
- SEKAR RAJESH BABU ET AL: "Fluorescence resonance energy transfer (FRET) microscopy imaging of live cell protein localizations." JOURNAL OF CELL BIOLOGY, vol. 160, no. 5, 3 March 2003 (2003-03-03) , pages 629-633, ISSN: 0021-9525
- LAKOWICZ J.R.: 'PRINCIPLES OF fLUORESCENCE SPECTROSCOPY', vol. 2 ED., 1999, KLUWER ACADEMIC/PLENUM PUBLISHERS, NEW YORK article LAKOWICZ, J.R.: 'Chapter 13 Energy Transfer', pages 367 - 389
- DUMY P. ET AL: 'A Convenient Synthesis of Cyclic Peptides as Regioselectively Addressable Functionalized Templates (RAFT)' TETRAHEDRON LETTERS, ELSEVIER, AMSTERDAM, NL LNKD- DOI:10.1016/0040-4039(94)02481-P vol. 36, no. 8, 20 Février 1995, pages 1255 - 1258, XP004028744 ISSN: 0040-4039
- SCHEIBLER L. ET AL: 'Functionalisation of gold surfaces via topological templates' TETRAHEDRON, ELSEVIER SCIENCE PUBLISHERS, AMSTERDAM, NL LNKD- DOI:10.1016/S0040-4020(98)00100-8 vol. 54, no. 15, 09 Avril 1998, pages 3725 - 3734, XP004111055 ISSN: 0040-4020
- SCHEIBLER L. ET AL: 'Functional Molecular Thin Films : Topological Templates for the Chemoselective Ligation of Antigenic Peptides to Self-Assembled Monolayers' ANGEWANDTE CHEMIE INTERNATIONAL EDITION ENGLISH vol. 38, no. 5, 1999, pages 696 - 699
- RENAUDET O. ET AL: 'Chemoselectively template-assembled glycoconjugates as mimics for multivalent presentation of carbohydrates' ORGANIC LETTERS, AMERICAN CHEMICAL SOCIETY, US LNKD- DOI:10.1021/OL0270935 vol. 5, no. 3, 01 Janvier 2003, pages 243 - 246, XP002251832 ISSN: 1523-7060
- RENAUDET O. ET AL: 'Chemoselectively template-assembled glycopeptide presenting clustered cancer related T-antigen' TETRAHEDRON LETTERS, ELSEVIER, AMSTERDAM, NL LNKD- DOI:10.1016/J.TETLET.2003.10.126 vol. 45, no. 1, 01 Janvier 2004, pages 65 - 68, XP004621813 ISSN: 0040-4039
- SINGH YASHVEER ET AL: "Preparation of a multitopic glycopeptide-oligonucleotide conjugate" ORGANIC LETTERS, AMERICAN CHEMICAL SOCIETY, US LNKD- DOI:10.1021/OL050134N, vol. 7, no. 7, 1 March 2005 (2005-03-01), pages 1359-1362, XP002491161 ISSN: 1523-7060
- GRIGALEVICIUS S. ET AL: 'Chemoselective assembly and immunological evaluation of multiepitopic glycoconjugates bearing clustered Tn antigen as synthetic anticancer vaccines' BIOCONJUGATE CHEMISTRY 200509 US LNKD- DOI:10.1021/BC050010V vol. 16, no. 5, Septembre 2005, pages 1149 - 1159 ISSN: 1043-1802
- BANFI DAMIANO ET AL: "Versatile synthesis of Boc protected hydrazinoacetic acid and its application to the chemoselective ligation of TASP molecules" PROTEIN AND PEPTIDE LETTERS, vol. 11, no. 6, December 2004 (2004-12), pages 539-542, ISSN: 0929-8665
- SCHATZLEIN ANDREAS G.: 'Targeting of synthetic gene delivery systems.' JOURNAL OF BIOMEDICINE AND BIOTECHNOLOGY vol. 2003, no. 2, 21 Avril 2003, pages 149 - 158 ISSN: 1110-7243
- SINGH YASHVEER ET AL: "Synthetic peptide templates for molecular recognition: Recent advances and applications" CHEMBIOCHEM, vol. 7, no. 9, September 2006 (2006-09), pages 1298-1314, ISSN: 1439-4227
- RAZKIN JESUS ET AL: "Activatable fluorescent probes for tumour-targeting imaging in live mice." CHEMMEDCHEM OCT 2006 LNKD- PUBMED:16944544, vol. 1, no. 10, October 2006 (2006-10), pages 1069-1072, ISSN: 1860-7179
- JIN ZHAO-HUI ET AL: "In vivo noninvasive optical imaging of receptor-mediated RGD internalization using self-quenched Cy5-labeled RAFT-c(-RGDfK-)(4)." MOLECULAR IMAGING : OFFICIAL JOURNAL OF THE SOCIETY FOR MOLECULAR IMAGING 2007 JAN-FEB LNKD- PUBMED:17311764, vol. 6, no. 1, January 2007 (2007-01), pages 43-55, ISSN: 1535-3508

## Description

La présente invention concerne le domaine de l'architecture de sondes moléculaires pour l'imagerie *in vivo*. Plus particulièrement, l'invention concerne des constructions moléculaires assurant une fonction d'imagerie par fluorescence activable en milieu intracellulaire, ces constructions étant en outre liées à un vecteur qui en permet le ciblage vers certaines cellules, et l'internalisation dans les cellules en question.

Le développement récent des méthodes optiques d'imagerie de fluorescence *in vivo* du petit animal ouvre de nouveaux horizons pour l'imagerie fonctionnelle. Il est maintenant possible de suivre en temps réel et de façon non invasive le devenir de molécules luminescentes, leur biodistribution, d'établir un diagnostic et d'évaluer l'effet d'une thérapeutique grâce à ces molécules. Les avantages de l'imagerie optique par rapport aux autres techniques d'imagerie fonctionnelle (IRM, PET, SPECT) sont les suivants :
- pas de manipulation de molécules radioactives et des contraintes qui y sont liées (radioprotection, gestion des déchets, source synchrotron pour les marqueurs PET),
- faible coût de l'instrumentation,
- bonne sensibilité par rapport à l'Imagerie par Résonance Magnétique (IRM) en termes de quantité de marqueur injectée.

Actuellement, l'imagerie optique fonctionnelle est essentiellement réalisée en utilisant des marqueurs luminescents greffés à un ligand biologique permettant de cibler certaines zones du sujet (organes, cellules, tumeurs...).

Différents marqueurs luminescents ont été développés pour ce type d'imagerie. En premier lieu, on peut citer les sondes à base de fluorophores organiques. Le premier marqueur utilisé, l'ICG (Indo Cyanin Green), a été employé très tôt « nu » (injection du fluorophore seul), pour imager/visualiser la vascularisation et la circulation dans les vaisseaux sanguins ; ces fluorophores organiques ont ensuite été greffés sur des protéines ou des anticorps pour cibler différentes cellules (Folli, Westerman et al. 1994 ; Neri, Carnemolla et al. 1997 ; Ballou, Fisher et al. 1995; Ballou, Fisher et al. 1998 ; Becker, Riefke et al. 2000). Cependant, le couplage à ces grosses molécules présente des inconvénients pour le ciblage et la pharmaco-cinétique (Bugaj, Achilefu et al. 2001) ; en conséquence, la fonctionnalisation de fluorophores par de petits peptides a récemment été préférée (Achilefu, Dorshow et al. 2000; Bugaj, Achilefu et al. 2001 ; Licha, Hessenius et al. 2001 ; Becker, Hessenius et al. 2001).

Un deuxième type de marqueurs pour l'imagerie de fluorescence *in vivo* est la classe des sondes à base de nanocristaux semi-conducteurs luminescents (Michalet, Pinaud et al. 2005). L'inconvénient majeur de ces marqueurs est leur mauvaise pharmaco-cinétique, qui nécessite un enrobage chimique spécial (Ballou, Lagerholm et al. 2004; Gao, Cui et al. 2004). Une seule publication est parue jusqu'à présent concernant de tels marqueurs luminescents fonctionnalisés pour cibler des tumeurs (Gao, Cui et al. 2004).

Les marqueurs présentés ci-dessus présentent toutefois une limitation majeure liée au signal non spécifique provenant des parties non ciblées du corps du sujet. En effet, les ligands biologiques existants ne permettent pas un ciblage à 100%, et la cinétique de ciblage peut être lente. De plus, pendant ce temps de fixation de la sonde sur son récepteur, celle-ci commence déjà à être métabolisée par l'organisme. Dans ces conditions, l'intervalle de temps optimal pour l'observation du processus biologique ciblé, c'est-à-dire l'intervalle de temps pendant lequel le contraste zone d'intérêt/reste du corps de l'animal est le plus grand, peut être court et difficile à déterminer. De plus, le contraste zone d'intérêt/reste du corps du sujet obtenu reste faible. Ce problème est encore plus crucial pour l'imagerie de fluorescence que pour les autres techniques d'imagerie, car la très importante diffusion de la lumière dans les tissus fait que le niveau du signal non spécifique est très élevé. Cela rend ainsi toute méthode tomographique de localisation des sondes difficile à mettre en oeuvre.

Pour améliorer le ciblage, certaines équipes ont proposé, plutôt que de greffer simplement le marqueur luminescent à un ligand biologique permettant de cibler la zone à imager, d'utiliser des vecteurs plus complexes. Ainsi, l'utilisation de nanoparticules, telles des nanoparticules de polymères (Weissleder, Tung et al. 1999; Bremer, Tuhg et al. 2001) ou des nanocristaux semi-conducteurs (jouant alors à la fois le rôle de vecteur et de marqueur luminescent, (Michalet, Pinaud et al. 2005)) commence à être répandue. Néanmoins, le greffage de différentes entités sur ces nanoparticules est complexe ; aussi, seules ont été proposées la fonctionalisation des nanocristaux semi-conducteurs luminescents par une entité de ciblage (Gao, Cui et al. 2004), et la fonctionalisation de particules de polymère par une fonction d'imagerie (Weissleder, Tung et al. 1999; Bremer, Tung et al. 2001). Josephson et al. (2002) décrit un vecteur comprenant une nanoparticule de type CLIO relié à un fluorophore par un pont disulfure et un bras peptidique.

Récemment, deux châssis moléculaires, susceptibles de constituer des alternatives aux nanoparticules pour porter des fonctions de ciblage et un marqueur pour l'imagerie de fluorescence, ont été décrits (Boturyn, Coll et al. 2004; Dumy, Favrot et al. 2004 ; Maison, Frangioni et al. 2004). Le premier, appelé RAFT (*Regioselectively Addressable Functionalized Template*), est un cyclodécapeptide ; le second est un dérivé de l'adamantane.

Une autre approche pour diminuer le bruit de fond lors de l'utilisation de sondes fluorescentes consiste à utiliser des sondes « activables » spécifiquement dans certaines cellules.

Le principe des sondes activables de première génération est d'utiliser un polymère PEG/poly-lysine comme vecteur (Weissleder, Tung et al. 1999). Un fluorophore Cy5.5 est lié au squelette du polymère par un bras pendant. Le rapport du nombre de fluorophores par unité de polymère est optimisé de telle façon que les Cy5.5 soient suffisamment proches pour que leur fluorescence soit auto-inhibée, et que la sonde soit initialement très faiblement fluorescente. Certaines enzymes, notamment surexprimées dans certains modèles de tumeurs, sont capables de couper le squelette du polymère. Sous l'action de ces enzymes, les fluorophores se retrouvent donc séparés et capables d'émettre. Par exemple, Weissleder et al. mesurent avec cette sonde un signal de fluorescence 80 fois plus élevé dans un modèle de tumeur que dans le sang, alors que la concentration en sondes y est 10 fois plus faible (Weissleder, Tung et al. 1999).

La deuxième génération des sondes activables adapte le principe pour l'imagerie spécifique d'une activité enzymatique protéolytique (*i.e*., capable de couper une protéine) (Bremer, Tung et al. 2001). Cette fois-ci, le bras pendant entre le squelette polymérique et le fluorophore est un bras peptidique, spécifique de l'activité enzymatique à imager. Ainsi, le peptide utilisé variera suivant que l'on souhaite imager l'activité de la MMP2 (Bremer, Tung et al. 2001), de la cathepsine D (Tung, Bredow et al. 1999; Tung, Mahmood et al. 2000), ou l'activation de la thrombine dans le sang (Tung, Gerszten et al. 2002).

Plus récemment, ce groupe a utilisé le principe non plus d'auto-inhibition de la fluorescence d'un même fluorophore, mais d'inhibition de la fluorescence d'un fluorophore par un autre inhibiteur (Pham, Choi et al. 2005). Ces processus photophysiques et photochimiques sont bien connus de l'homme du métier et sont décrits par exemple dans l'ouvrage de J. Lakowicz (Lakowicz 1999). Ils sont utilisés pour imager une activité enzymatique : après clivage enzymatique du bras peptidique, le fluorophore et son inhibiteur sont séparés et le fluorophore peut alors émettre (Pham, Choi et al. 2005).

Les sondes proposées dans ces travaux sont donc des sondes non fluorescentes initialement, et dont la fluorescence se déclenche uniquement dans les zones où existe l'activité enzymatique pour l'imagerie de laquelle elles ont été architecturées. Cependant, ces sondes présentent les inconvénients suivants :
- elles sont limitées à imager une activité enzymatique, et le principe utilisé ne peut être élargi pour détecter d'autres processus biologiques, tels que la reconnaissance d'un récepteur par un ligand,
- un bras peptidique clivable enzymatiquement doit être déterminé pour chaque enzyme dont on souhaite imager l'activité,
- ces sondes ne vont pas *a priori* dans des zones localisées de l'animal, et ainsi peuvent conduire à un signal non voulu. Par exemple, les sondes pour l'imagerie de l'activité enzymatique de la MMP2 ou de la cathepsine D ont été utilisées pour imager des tumeurs surexprimant ces enzymes. Néanmoins, le bras peptidique de ces sondes peut être coupé par d'autres protéases. En outre, en l'absence de ciblage, la quantité de sondes à utiliser est importante.

Il existe donc aujourd'hui un réel besoin d'un vecteur d'imagerie ciblé, qui soit tel que le bruit de fond dans les régions non ciblées soit nul ou quasi-nul, et que l'intensité du signal d'émission, au niveau de la zone de ciblage, soit indépendante d'une activité enzymatique spécifique (présente uniquement dans certains types cellulaires, ou de niveau variable selon les cellules), afin d'obtenir une image reflétant fidèlement le ciblage lui-même. Le but de la présente invention est de fournir des outils d'imagerie remédiant à au moins une partie des inconvénients des vecteurs actuels, tels qu'exposés ci-dessus.

Pour cela, les inventeurs ont mis au point un système moléculaire permettant l'activation d'une fonction d'imagerie dans le milieu intracellulaire de toute cellule eucaryote. Le couplage d'un tel système à un vecteur ciblé qui est internalisé dans les cellules cibles, permet que le signal d'émission ne se déclenche qu'après qu'une cellule cible ait été atteinte.

Un premier objet de l'invention est donc un vecteur biologique ciblé, comprenant un cyclodécapeptide RAFT, auquel est liée une fonction d'imagerie activable dans le milieu intracellulaire, et tel que la fonction d'imagerie est activée par un mécanisme ubiquitaire. Par « mécanisme ubiquitaire », on entend ici un mécanisme qui se produit dans le milieu intracellulaire de toute cellule eucaryote. Dans ce qui suit, une « fonction d'imagerie » désignera la fonction elle-même, mais également, par abus de langage, les moyens (fluorophores, particules luminescentes, ...) permettant d'obtenir cette fonction. De même, une « fonction de ciblage » peut désigner les moyens utilisés pour le ciblage (ligands, anticorps, ...). Un vecteur biologique ciblé comportant une fonction d'imagerie peut également être appelé « sonde ».

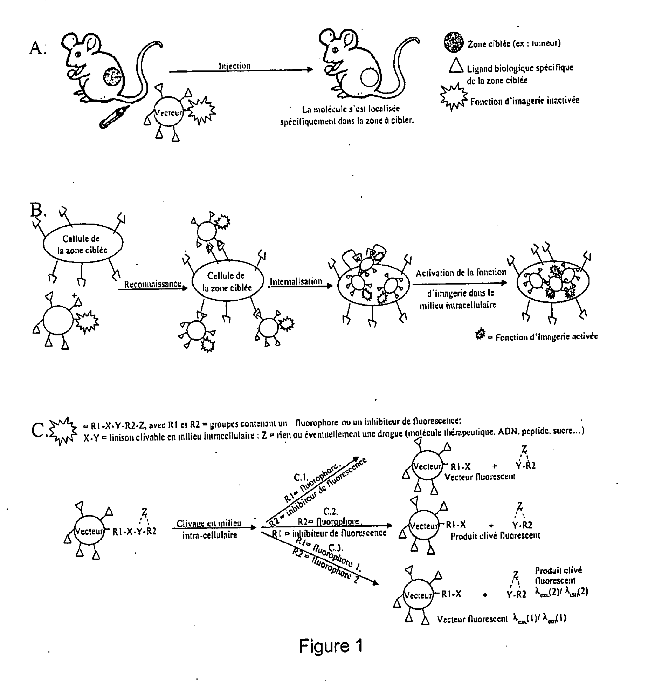

Selon l'invention, la fonction d'imagerie activable est assurée par un fluorophore F relié à un inhibiteur de fluorescence par un bras clivable dans le milieu intracellulaire. L'internalisation du vecteur, suite à sa liaison à la cellule cible, entraînera le clivage du bras reliant l'inhibiteur de fluorescence au fluorophore, et donc l'activation de la fluorescence. La figure 1 illustre ce mode de réalisation de l'invention. La sonde selon l'invention y est représentée par la formule « Vecteur-R1-X-Y-R2 », où « Vecteur » désigne le vecteur ciblé, R1 et R2 sont des groupes contenant chacun un fluorophore ou un inhibiteur de fluorescence, et X-Y représente la liaison clivable en milieu intracellulaire. Un groupement Z, symbolisant une drogue (molécule thérapeutique par exemple) est, le cas échéant, fixé à Y et/ou à R2. En outre, les groupes R1 et R2 peuvent comprendre un groupe chimique de liaison permettant l'attache dudit fluorophore ou inhibiteur de fluorescence au vecteur ou au bras clivé (ou éventuellement à la drogue Z), et, le cas échéant, un espaceur chimique permettant de diminuer les interactions stériques entre le vecteur et/ou le bras clivé d'une part, et le fluorophore et/ou l'inhibiteur de fluorescence d'autre part. Dans la suite du présent texte, les notations R1, R2, X, Y et Z conservent cette signification.

A titre d'exemples de fluorophores F utilisables, on peut citer :
- un fluorophore organique : de nombreux fluorophores sont commercialisés chez différents fournisseurs (Sigma-Aldrich, Molecular Probes, FluoProbes...). Ces fluorophores peuvent être, entre autres, des fluorescéines, des coumarines, des bodipys, des porphyrines, des cyanines, des rhodamines, des oxazines. Cette liste n'est pas exhaustive.
- des nanoparticules (nanocristaux semi-conducteurs (quantum dots), nanoparticules d'or, nanoparticules à base de polymères, nanoparticules d'oxydes...) ayant des propriétés d'émission. Cette liste n'est pas exhaustive.

Ces fluorophores peuvent être liés au vecteur et/ou au bras clivable notamment par des fonctions amide, ester, thioéther ou thioester. Comme mentionné ci-dessus, un bras espaceur entre le vecteur et/ou le bras clivable et le fluorophore peut être également présent, notamment pour diminuer une éventuelle inhibition de la fluorescence du fluorophore par le vecteur et/ou le bras clivable.

A titre d'inhibiteurs de fluorescence non fluorescents, on peut citer :
- une molécule organique : Dabcyl et dérivés, famille des BHQ (« Black Hole Quencher », Biosearch Technologies), famille des QSY (Molecular Probes), ou encore la famille des CyQ (Amersham). Cette liste n'est pas exhaustive. Deux fournisseurs au moins proposent de telles molécules commercialement : Biosearch Technologies et Molecular Probes, Amersham Biosciences.
- des nanoparticules telles des nanocristaux semi-conducteurs (« quantum dots »), des nanoparticules d'or, des nanoparticules à base de polymères, des nanoparticules d'oxydes. Cette liste n'est pas exhaustive.

Ces inhibiteurs de fluorescence peuvent être liés au vecteur et/ou au bras clivable par des fonctions amide, ester, thioéther, thioester ou autre. Un bras espaceur entre le vecteur et/ou le bras clivable et l'inhibiteur de fluorescence peut être également présent.

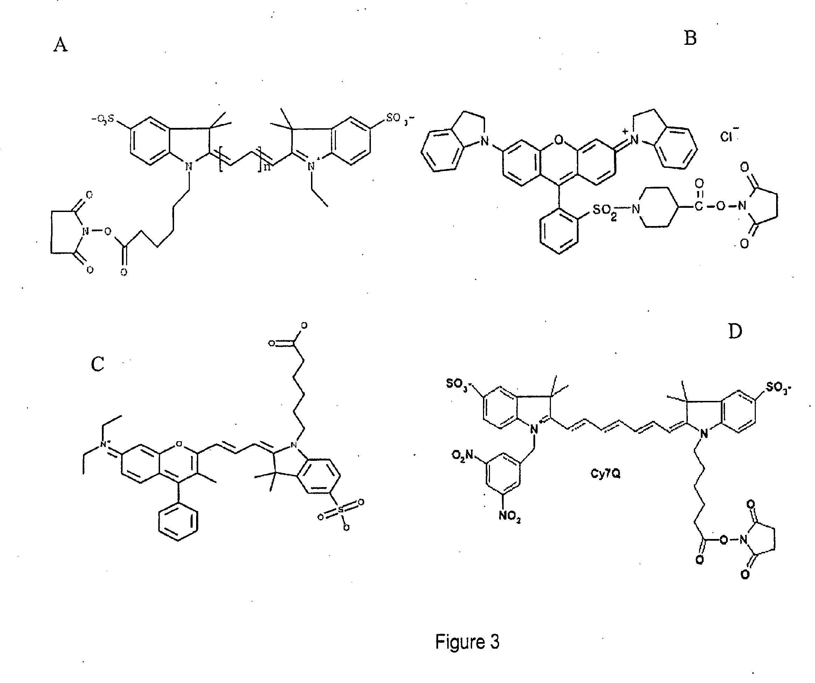

Quelques exemples non limitatifs de structures de fluorophores et d'inhibiteurs de fluorescence sont représentés à la figure 3.

Le bras reliant l'inhibiteur de fluorescence et le fluorophore est de préférence clivable dans le milieu intracellulaire de toute cellule. Le clivage de la liaison X-Y peut être par exemple une réaction acido-basique, redox, organométallique, catalysée par une enzyme ou non. Selon l'invention, le bras clivable en milieu intracellulaire comporte un pont disulfure. Un exemple de bras pouvant être utilisé comporte un pont disulfure entre deux cystéines (X=Y=Cys, la liaison entre les deux cystéine étant une liaison S-S et non une liaison peptidique, la liaison X-Y étant alors notée Cys-S-S-Cys). Un autre exemple est un bras qui est simplement constitué de deux atomes de soufre (X=Y=S). Lorsque le bras clivable comprend un pont disulfure, le clivage est catalysé enzymatiquement par les thiorédoxines, dans les lysosomes et endosomes des cellules (Arunachalam, Phan et al. 2000). Les thiorédoxines sont des petites protéines impliquées dans la régulation redox intracellulaire, et donc dans de nombreux processus fondamentaux (réponse au stress, apoptose...). Elles réduisent tous les ponts disulfure des protéines entrant dans la cellule, mais ne détruisent pas la liaison peptidique entre les acides aminés, contrairement aux protéases. Elles sont présentes dans toutes les cellules de tous les organismes.

Selon un mode de réalisation préféré des vecteurs selon l'invention, le ciblage est assuré par au moins un ligand biologique reconnu par un récepteur surexprimé à la surface de certaines cellules. Les ligands biologiques permettant de cibler spécifiquement certaines cellules peuvent être :
- des peptides, par exemple le peptide RGD, ou leurs dérivés ou leurs analogues (ex : le peptide octéotrate, analogue de la somatostatine, un analogue de la bombésine, de la neurotensine, l'EGF, le VIP...),
- des protéines, des anticorps, ou leurs dérivés ou leurs analogues,
- des sucres, notamment des monosaccharides (ex : glucose, galactose, glucosamine ou galactosamine), des oligosaccharides, des polysaccharides, ou leurs dérivés ou leurs analogues,
- des oligonucléotides, ADN, ARN, leurs dérivés ou leurs analogues,
- des molécules organiques (telles que le folate ou le pamidronate biphosphonaté),
- des complexes organométalliques.

Leur activité de ciblage est due à la reconnaissance moléculaire de ces ligands par des récepteurs surexprimés à la surface des cellules de la zone d'intérêt.

Des ligands particulièrement préférés pour mettre en oeuvre l'invention sont par exemple des peptides comportant le motif RGD, tels que le cyclo(RGDfK), le cyclo(RGDyK) ou le cyclo(RGDfV). Ces peptides reconnaissent l'intégrine αᵥβ₃, qui est surexprimée à la surface des cellules tumorales et des cellules endothéliales lors de la néoangiogénèse tumorale. L'utilisation de ces ligands dans les vecteurs selon l'invention permet donc d'imager les tumeurs et leur vascularisation - et, le cas échéant, de délivrer une drogue à ce niveau. Un autre ligand préféré est par exemple un peptide comportant le motif NGR décrit par Curnis *et al*. (Curnis, Arrigoni et al. 2002), lequel cible également les néo-vaisseaux.

Selon un mode de réalisation particulier de l'invention, le vecteur peut comporter un ou plusieurs marqueur(s) pour une autre modalité d'imagerie que l'imagerie de fluorescence, en plus de cette dernière. Ces marqueurs peuvent être greffés à la surface du vecteur. De tels marqueurs peuvent être :
- des chélates de gadolinium, des nanoparticules d'oxyde de fer, ou d'autres agents de contraste pour l'IRM, connus de l'homme du métier ;
- des molécules radiomarquées, par exemple par du ⁹⁹Tc, du ¹¹¹In, du ¹⁸F, du ¹¹C ou du ¹⁵O, utilisés comme agents de contraste pour l'imagerie nucléaire et connus de l'homme du métier.

Selon un mode de réalisation particulier de l'invention, le vecteur est en outre capable de délivrer une drogue Z. Cette drogue peut être accrochée au vecteur ou au bras clivable par différents groupes chimiques (par exemple : fonctions acide, ester, thioéther ou thioester) via éventuellement un bras espaceur. Elle peut également être liée au vecteur par un bras clivable tel que les bras X-Y précédemment décrits, ou par un autre bras clivable chimiquement ou clivable par un autre processus (activation lumineuse, ultrasons, radio fréquence...). Lorsque la drogue est soit reliée au vecteur par le même bras clivable que celui contenu dans la fonction d'imagerie, soit elle-même marquée par le fluorophore ou l'inhibiteur de fluorescence de la fonction d'imagerie, l'activation de la fonction d'imagerie démontre également la délivrance de la drogue.

Par « drogue », on entend ici toute molécule susceptible d'avoir un effet sur la cellule dans laquelle elle sera délivrée. Cet effet est de préférence thérapeutique pour le sujet auquel elle est administrée. A titre d'exemples de drogues Z utilisables dans le cadre de l'invention, on peut citer :
- une molécule thérapeutique déjà identifiée comme telle (exemple : taxol, doxorubicine, paclitaxel...)
- un ADN ou un oligonucléotide naturel ou synthétique, par exemple un siARN destiné à inhiber la synthèse d'une protéine,
- un peptide ou une protéine naturel(le) ou synthétique (obtenu suivant les techniques de l'homme du métier, par digestion enzymatique ou par synthèse sur support solide par exemple),
- un monosaccharide, oligosaccharide ou polysaccharide ou dérivés et analogues.

Selon un mode de réalisation particulier de l'invention, la fonction d'imagerie activable est assurée par un fluorophore F relié à un inhibiteur de fluorescence par un bras clivable dans le milieu intracellulaire, de telle sorte qu'après clivage dudit bras clivable, l'inhibiteur reste lié au vecteur et le fluorophore reste lié à la drogue Z. Suivant la notation utilisée dans la figure 1 et explicitée ci-dessus, cela implique que la drogue Z est liée au groupe R2 et/ou à Y.

Plusieurs configurations de ce mode de réalisation, ou du mode de réalisation « Vecteur-R1-X-Y-R2 » en l'absence de drogue Z, peuvent être envisagées et aisément mises en oeuvre par l'homme du métier, suivant l'application choisie. Ces différentes configurations sont illustrées à la figure 1C, et sont parties intégrantes de l'invention.

Dans le cas illustré à la figure 1C.1, le fluorophore F est sur le groupe R1, et R2 comprend un inhibiteur non fluorescent de la fluorescence de F (R2 peut être limité à cet inhibiteur). On obtient alors, après activation de la fonction d'imagerie, un marquage fluorescent du vecteur, qui permet de visualiser le ciblage de la sonde et de suivre la biodistribution du vecteur lui-même.

La figure 1C.2 il.lustre le cas où le fluorophore F est sur le groupe R2, et où un inhibiteur non fluorescent de la fluorescence de F est sur R1. On obtient alors, après activation de la fonction d'imagerie, un marquage fluorescent du produit clivé R2-Y ou R2-Y-Z ou Y-R2-Z, qui permet de visualiser le ciblage de la sonde et de suivre la biodistribution du produit clivé et donc, le cas échéant, de la drogue Z délivrée.

Dans le cas où le même fluorophore F est sur le groupe R1 et sur le groupe R2, F doit être un fluorophore dont la fluorescence peut s'auto-inhiber. C'est le cas par exemple des fluorophores de la famille des cyanines. L'activation de la fonction d'imagerie permet alors de visualiser le ciblage de la sonde et de suivre le vecteur et le produit clivé, sans distinction. La fluorescence libérée est dans ce cas potentiellement deux fois plus importante que dans les cas exposés ci-dessus et illustrés à la figure 1C.1 et 1C.2.

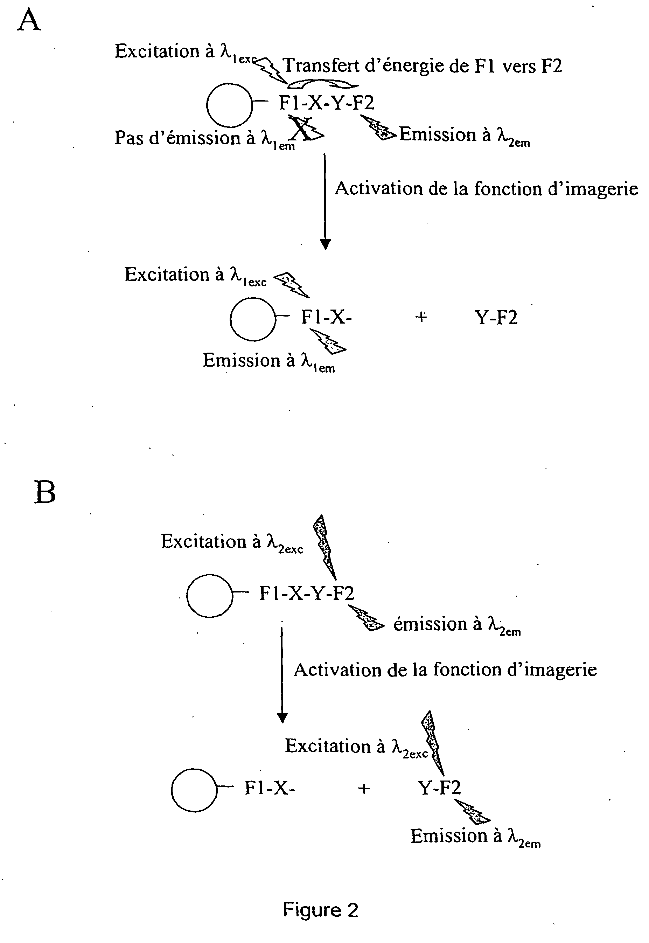

Il est également possible d'utiliser, comme inhibiteur de fluorescence, un deuxième fluorophore. Dans ce cas, les deux fluorophores F1 et F2 (avec F1 ≠ F2) sont choisis tels que le fluorophore F1, excité à sa longueur d'onde d'excitation λ_{exc}(1), a initialement sa fluorescence inhibée par le fluorophore F2 par le processus de transfert d'énergie bien connu de l'homme du métier (Lakowicz 1999). Il n'y a alors initialement qu'un signal d'émission à la longueur d'onde d'émission de F2, λₑₘ(2), et non à la longueur d'onde d'émission de F1, λₑₘ(1) (Figure 2). L'activation de l'activité d'imagerie se traduit par l'émission d'un'signal à la longueur d'onde d'émission de F1, λₑₘ(1) lors de l'excitation à λ_{exc}(1).

Dans le cas, illustré à la figure 1C.3, où le fluorophore F1 est sur le groupe R1 et le fluorophore F2 est sur le groupe R2, ce signal indique le ciblage de la sonde et permet de visualiser la biodistribution du vecteur en excitant le système à la longueur d'onde d'excitation de F1 λ_{exc}(1) et en détectant le signal à la longueur d'onde d'émission de F1 λₑₘ(1), et de visualiser la biodistribution du produit clivé en excitant le système à la longueur d'onde d'excitation de F2 λ_{exc}(2) et en détectant le signal à la longueur d'onde d'émission de F2 λₑₘ(2) (Figure 2). L'excitation à la longueur d'onde d'émission de F1 λ_{exc}(1), et la détection à λₑₘ(2), permet en outre de suivre la biodistribution de la sonde avant l'activation de la fonction d'imagerie (Figure 2). Une telle configuration présente donc l'avantage de suivre à la fois la biodistribution de la sonde (en excitant à λ_{exc}(2), et en observant à λₑₘ(2)), et l'activation de la fonction d'imagerie (en excitant à λ_{exc}(1), et en observant à λₑₘ(1)) (Figure 2). C'est donc l'une des configurations les plus avantageuses de l'invention.

Les rôles de F1 et F2 peuvent être inversés, à savoir que F1 et F2 peuvent être choisis tels que le fluorophore F2, excité à sa longueur d'onde d'excitation λ_{exc}(2), a initialement sa fluorescence inhibée par le fluorophore F1. L'activation de la fonction d'imagerie conduira au même résultat, à savoir visualiser le ciblage de la sonde, et visualiser la biodistribution du vecteur en excitant le système à la longueur d'onde d'excitation de F1 λ_{exc}(1) et en détectant le signal à la longueur d'onde d'émission de F1 λₑₘ(1), et visualiser la biodistribution du produit clivé en excitant le système à la longueur d'onde d'excitation de F2 λ_{exc}(2) et en détectant le signal à la longueur d'onde d'émission de F2 λₑₘ(2).

L'invention porte donc également sur l'utilisation d'un vecteur tel que décrit ci-dessus (configuration avec deux fluorophores F1 et F2), pour délivrer une drogue, par exemple une molécule thérapeutique, et suivre simultanément la biodistribution du vecteur et de la drogue en question. Cette configuration, ainsi que celle où R2 comprend un fluorophore F et R1 un inhibiteur pur de fluorescence, peuvent aussi être utilisées avantageusement pour suivre en temps réel la biodistribution d'une drogue. Par « inhibiteur pur », on entend ici un inhibiteur de fluorescence qui est lui-même non fluorescent.

De préférence, un vecteur selon la présente invention est électriquement neutre avant activation de la fonction imagerie. En effet, une molécule neutre pénètre plus facilement dans la cellule qu'une molécule chargée. Ainsi, dans les exemples qui suivent, le vecteur RAFT-Cy5-Cys-S-S-Cys-Q pénètre mieux dans les cellules que le vecteur RAFT-Cy5-Cys-S-S-Cys-Cy5. Il est par ailleurs avantageux que les deux molécules issues du clivage du bras X-Y soient chargées, car les molécules chargées restent plus facilement dans le milieu intracellulaire. Ainsi, dans l'exemple décrit plus loin, le clivage du pont disulfure du vecteur RAFT-Cy5-Cys-S-S-Cys-Q (RAFT étant en outre lié à un ligand neutre assurant le ciblage, et l'inhibiteur Q pouvant être couplé à une drogue électriquement neutre) donne deux entités moléculaires de charges opposées.

Selon un mode de réalisation préféré des vecteurs de l'invention, la fonction d'imagerie est donc assurée par une cyanine, par exemple le fluorophore Cy5, relié par un pont disulfure à un inhibiteur de sa fluorescence.

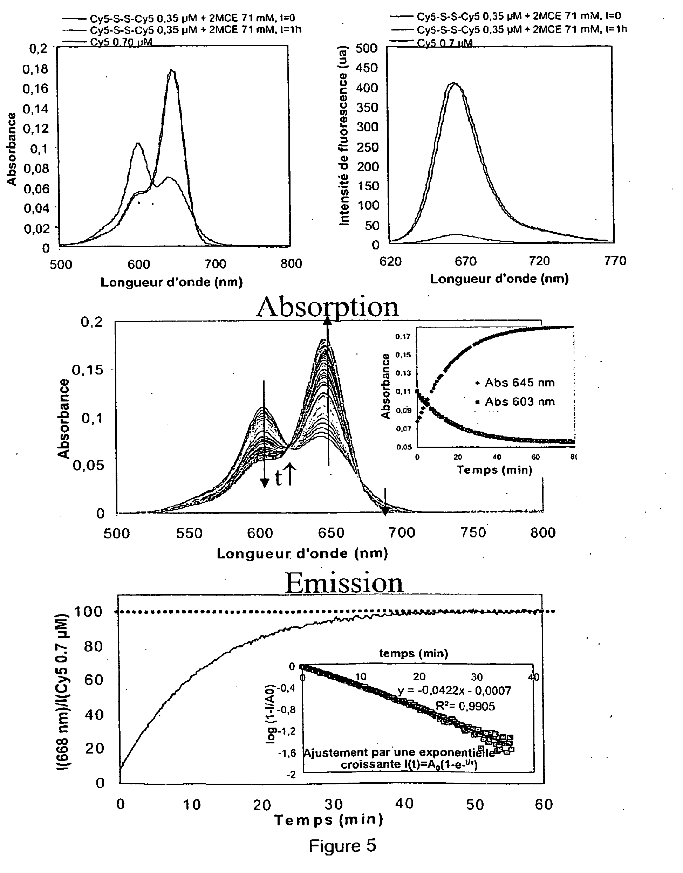

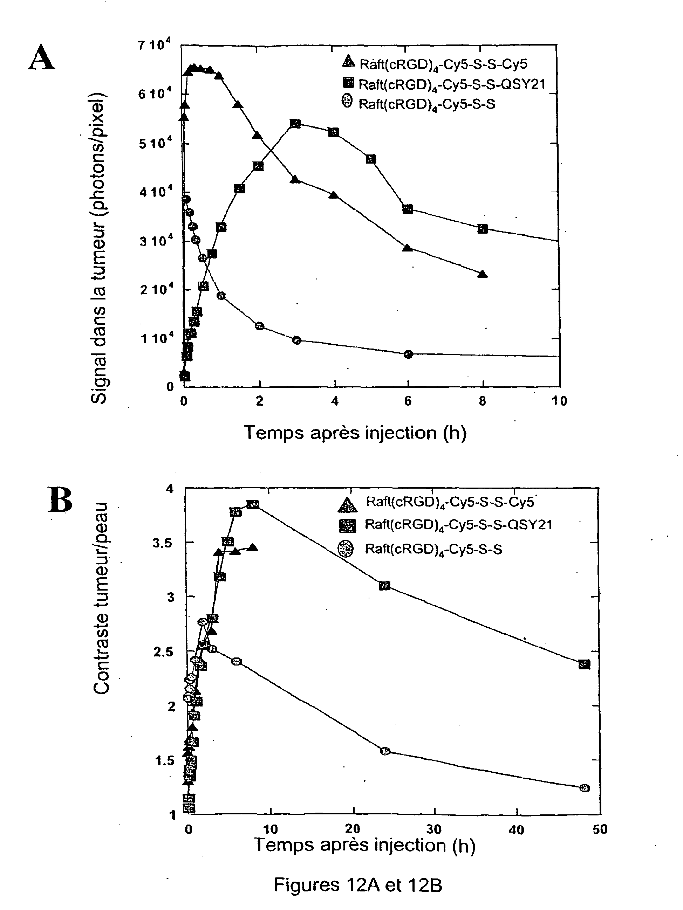

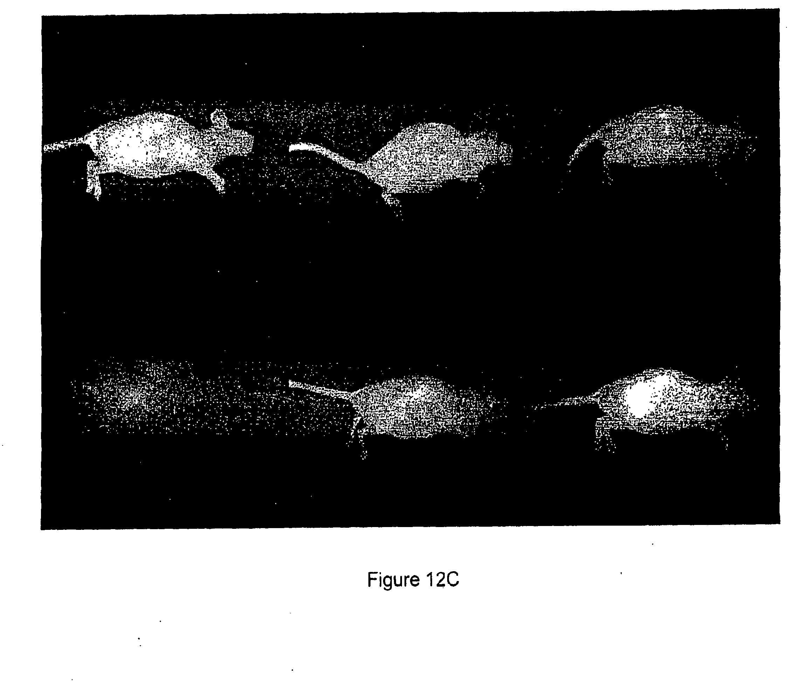

Les résultats présentés dans la partie expérimentale ci-dessous montrent qu'un vecteur selon l'invention possède des propriétés pharmacocinétiques telles que la réponse en imagerie, au niveau de la zone ciblée, est croissante dans le temps (figure 12), au moins pendant la première heure suivant l'injection du vecteur. Ceci est lié aux moins en partie au fait que l'activation de la fluorescence a lieu dans le milieu intracellulaire, donc uniquement après que le vecteur ait atteint une cellule cible et ait été internalisé. L'utilisation d'un pont disulfure, de telle sorte que le clivage de la liaison S-S entraîne l'activation de la fonction imagerie, permet une bonne cinétique d'apparition du signal. Les figures 5, 6, 10 et 11 montrent que la cinétique de clivage chimique du pont disulfure par le 2-mercaptoéthanol (2-MCE) est lente, malgré la concentration élevée en 2-MCE. Le clivage est encore plus lent dans la cellule, car la concentration en enzyme est faible. Ceci entraîne une montée lente de l'intensité du signal, qui demeure longtemps et permet l'élimination de la sonde non-ciblée pendant ce temps, ce qui favorise la spécificité. Cette propriété des vecteurs de l'invention est particulièrement avantageuse, car elle donne au praticien une plus grande liberté de manoeuvre pour acquérir l'image - spécifique de la zone ciblée - dans de bonnes conditions. Cette propriété des vecteurs décrits ici est donc un aspect important de l'invention.

Les exemples et figures présentés ci-dessous à titre non limitatif permettront de mettre en évidence certains avantages et caractéristiques, ainsi que d'autres dispositions avantageuses de la présente invention.

### LEGENDE DES FIGURES:

**Figure 1** **:** cette figure décrit le principe de l'invention. La sonde comporte initialement au moins un ligand biologique pour le ciblage (elle peut en comporter plusieurs), et une fonction d'imagerie inactivée. Elle peut en outre comporter une fonction de délivrance de drogue.
   La **figure 1A** décrit le principe du ciblage de la zone du sujet à imager par l'objet de l'invention. Le ciblage est obtenu par la fonctionnalisation du vecteur par un ligand biologique spécifique des cellules de la zone à cibler.
   La **figure 1B** décrit le processus conduisant à l'activation de la fonction d'imagerie de l'objet de l'invention. Après reconnaissance moléculaire entre les cellules de la zone ciblée et le ligand biologique porté par le vecteur, la sonde est internalisée dans les cellules. L'internalisation de la sonde dans les cellules déclenche l'activation de la fonction d'imagerie (*i.e*., la fluorescence).
   La **figure 1C** décrit la fonction d'imagerie. Celle-ci comprend au moins un fluorophore F (soit sur le groupe R1, soit sur le groupe R2), un bras X-Y clivable enzymatiquement en milieu intracellulaire, et un inhibiteur de fluorescence (soit sur le groupe R1, soit sur le groupe R2). Ce dernier peut-être soit un inhibiteur de fluorescence non fluorescent, soit un autre fluorophore. Dans ce dernier cas, il peut s'agir du même fluorophore que F (si F est un fluorophore s'auto-inhibant), ou d'un autre fluorophore pouvant absorber le rayonnement émis par F et le réémettre à une autre longueur d'onde. Cette fonction d'imagerie peut en outre comprendre une autre fonctionnalité, telle que la présence d'une drogue Z, de préférence greffée sur la partie clivable de la sonde (*i.e*., Y-R2). Dans le milieu intracellulaire, le bras clivable X-Y est coupé, séparant le vecteur lié au groupe R1-X, du produit R2-Y (ou R2-Y-Z ou Y-R2-Z).
**Figure 2** **:** Cas où le fluorophore F1 est sur le groupe R1 et le fluorophore F2 est sur le groupe R2, F1 ≠ F2, F1 et F2 choisis tels que le fluorophore F1, excité à sa longueur d'onde d'excitation λ_{exc}(1), a initialement sa fluorescence inhibée par le fluorophore F2 par le processus de transfert d'énergie bien connu de l'homme du métier (Lakowicz 1999). Il n'y a alors initialement qu'un signal d'émission à la longueur d'onde d'émission de F2, λₑₘ(2), et non à la longueur d'onde d'émission de F1, λₑₘ (1). L'activation de l'activité d'imagerie se traduit par l'émission d'un signal à la longueur d'onde d'émission de F1, λₑₘ(1). Ce signal indique le ciblage de la sonde et permet de visualiser la biodistribution du vecteur en excitant le système à la longueur d'onde d'excitation de F1 λ_{exc}(1) et en détectant le signal à la longueur d'onde d'émission de F1 λₑₘ(1), et de visualiser la biodistribution du produit clivé en excitant le système à la longueur d'onde d'excitation de F2 λ_{exc}(2) et en détectant le signal à la longueur d'onde d'émission de F2 λₑₘ(2).
**Figure 3** **:** Structure de quelques fluorophores et inhibiteurs (quenchers) commerciaux pouvant être utilisés pour réaliser l'objet de l'invention. **A :** Fluorophore : Cy5 (n=2) ou Cy7 (n=3) ester N-hydroxysuccinimidyl, Amersham. **B :** Quencher : QSY™ 21 ester N-hydroxysuccinimidyl, Molecular Probes. **C :** Fluorophore : NIR 700 - acide carboxylique, Fluka. **D :** Quencher : Cy7Q - ester N-hydroxysuccinimidyl, Amersham/GE Healthcare. Cette figure n'est pas exhaustive des composés utilisables selon l'invention.

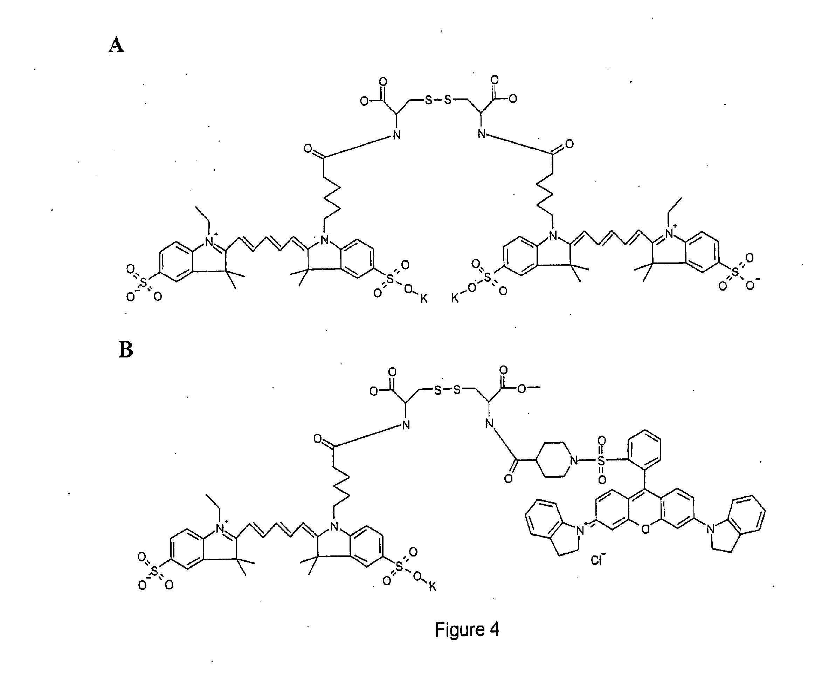

**Figure 4** **:** Structure chimique des fonctions d'imagerie Cy5-Cys-S-S-Cys-Cy5 (A) et Cy5-Cys-S-S-Cys-QSY21 (B).
**Figure 5** **:** Absorption et fluorescence de la fonction d'imagerie Cy5-Cys-S-S-Cys-Cy5 avant et après clivage chimique du pont disulfure par le 2-mercaptoéthanol (2-MCE). **A.** Spectre d'absorption et son évolution temporelle lors de l'ajout de 2-MCE. **B.** Mise en évidence que l'ajout de 2-MCE conduit au recouvrement total du spectre d'absorption de Cy5. **C.** Evolution temporelle de la fluorescence de la fonction d'imagerie lors de l'ajout de 2-MCE. D. Mise en évidence que l'ajout de 2-MCE conduit au recouvrement total de la fluorescence de Cy5. (fonction d'imagerie à 0.35 µM dans du Tris-HCl 10 mM pH 7.5, [2-MCE] = 70 mM).

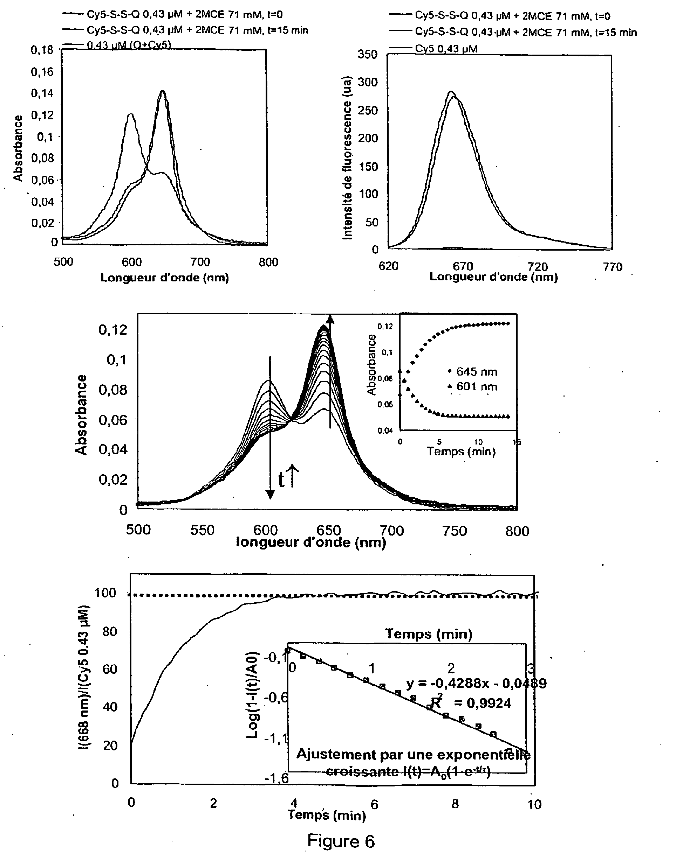

**Figure 6** **:** Absorption et fluorescence de la fonction d'imagerie Cy5-Cys-S-S-Cys-QSY21 avant et après clivage chimique du pont disulfure par le 2-mercaptoéthanol (2-MCE). **A.** Spectres d'absorption et leur évolution temporelle lors de l'ajout de 2-MCE. **B.** Mise en évidence que l'ajout de 2-MCE conduit au recouvrement total du spectre d'absorption de Cy5 et QSY21. **C.** Evolution temporelle de la fluorescence de la fonction d'imagerie lors de l'ajout de 2-MCE. **D.** Mise en évidence que l'ajout de 2-MCE conduit au recouvrement total de la fluorescence de Cy5. (fonction d'imagerie à 0.35 µM dans du Tris-HCl 10 mM pH 7.5, [2-MCE] = 70 mM).

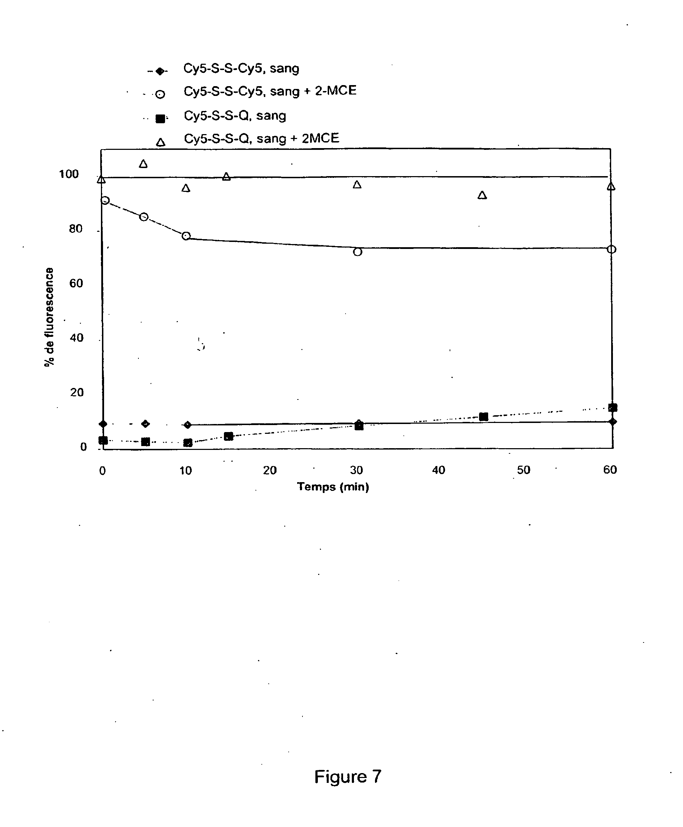

**Figure 7** **:** Evolution de la fluorescence des fonctions d'imagerie Cy5-Cys-S-S-Cys-Cy5 et Cy5-Cys-S-S-Cys-QSY21 ≈ 0.4 µM incubées dans le sang de souris. Les symboles pleins représentent la fluorescence mesurée dans le surnageant juste après centrifugation des échantillons prélevés au bout de différents intervalles de temps d'incubation. Les symboles vides représentent la fluorescence mesurée sur ces mêmes échantillons, 1h après ajout de 2-mercpatoéthanol (70 mM). Ces dernières courbes permettent donc d'évaluer la proportion de la fonction d'imagerie adsorbée sur les protéines sanguines.

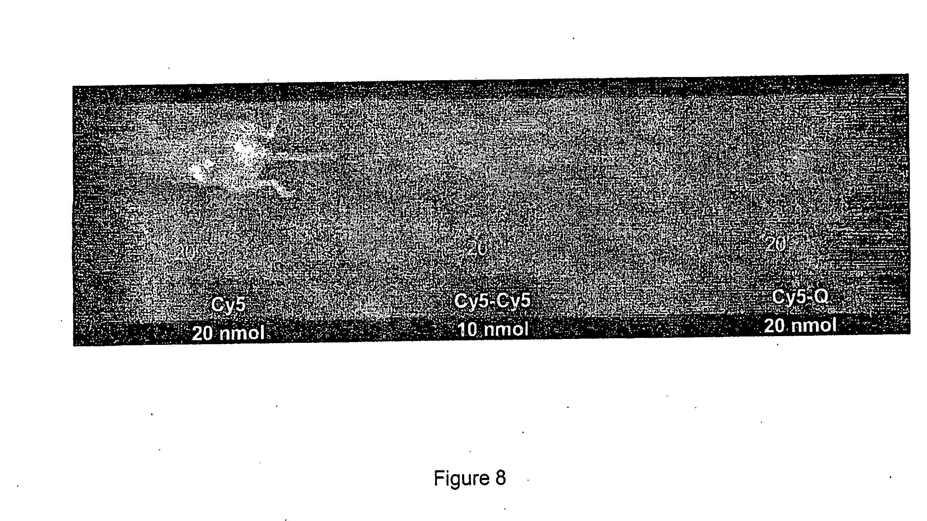

**Figure 8** **:** Fluorescence des fonctions d'imagerie Cy5-Cys-S-S-Cys-Cy5 et Cy5-Cys-S-S-Cys-QSY21 20 minutes après injection par voie intraveineuse dans des souris nude anesthésiées. L'acquisition des images se fait avec un dispositif d'imagerie de fluorescence par réflectance (FRI = Fluorescence Reflectance Imaging), comportant comme source d'excitation une couronne de LEDs munies de filtres interférentiels, émettant à 633 nm (puissance d'éclairement 50 µW.cm⁻²). Les images sont recueillies après filtration par un filtre coloré RG665 de densité optique >5 à la longueur d'onde d'excitation par une caméra CCD (Orca BTL, Hamamatsu) avec un temps d'exposition de 100 ms.

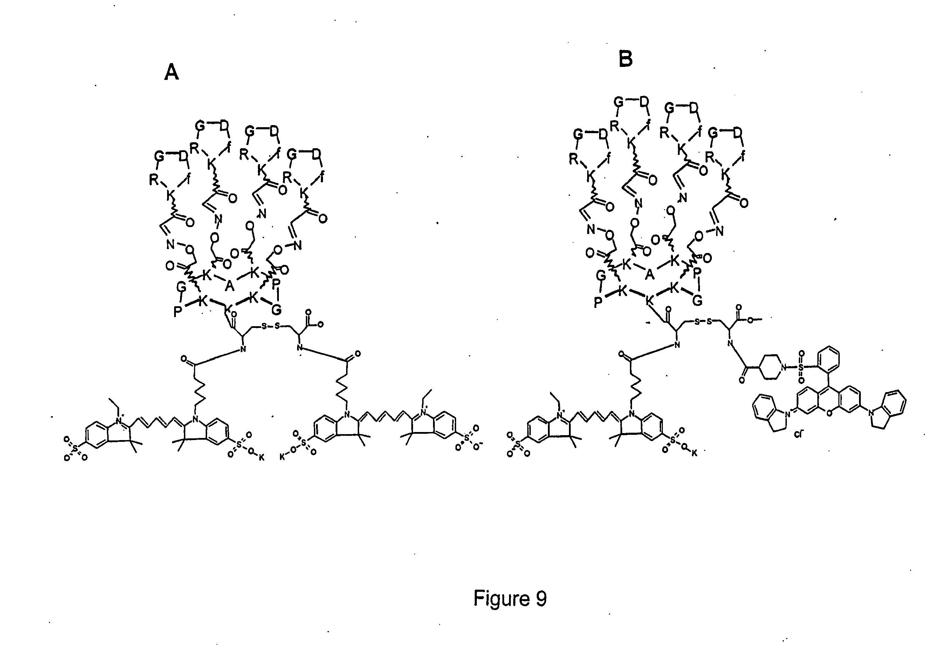

**Figure 9** **:** Structure chimique des molécules RAFT-(cRGD)₄-F où F représente les fonctions d'imagerie Cy5-Cys-S-S-Cys-Cy5 (A) et Cy5-Cys-S-S-Cys-QSY21 (B).

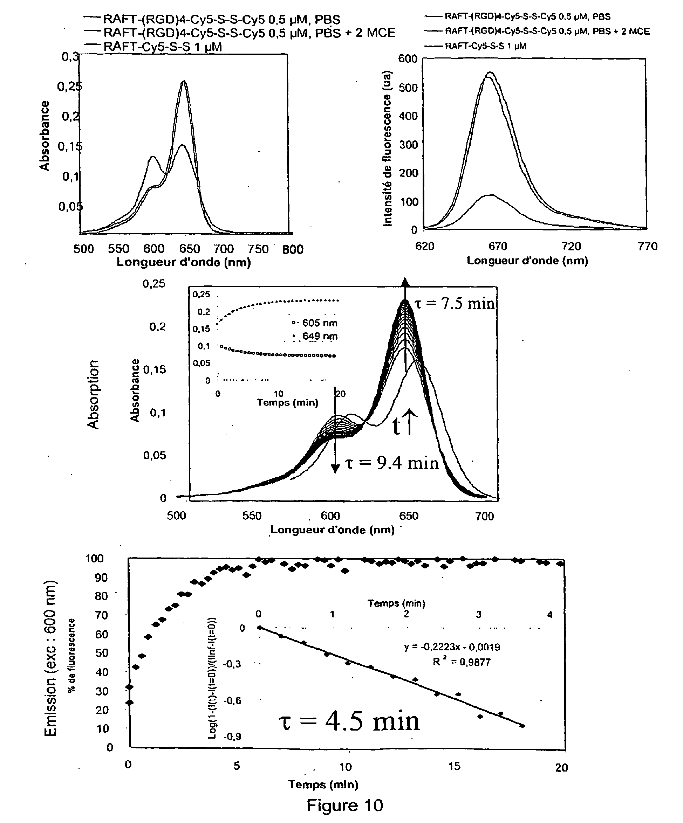

**Figure 10** **:** Absorption et fluorescence de la fonction d'imagerie Cy5-Cys-S-S-Cys-Cy5 greffée sur le RAFT-(cRGD)₄ avant et après clivage chimique du pont disulfure par le 2-mercaptoéthanol (2-MCE). **A.** Spectre d'absorption et son évolution temporelle lors de l'ajout de 2-MCE. **B.** Mise en évidence que l'ajout de 2-MCE conduit au recouvrement total du spectre d'absorption de Cy5. C. Evolution temporelle de la fluorescence de la fonction d'imagerie lors de l'ajout de 2-MCE. D. Mise en évidence que l'ajout de 2-MCE conduit au recouvrement total de la fluorescence de Cy5. (fonction d'imagerie à 0.5 µM dans du PBS 10 mM pH 7.2, [2-MCE] = 85 mM).

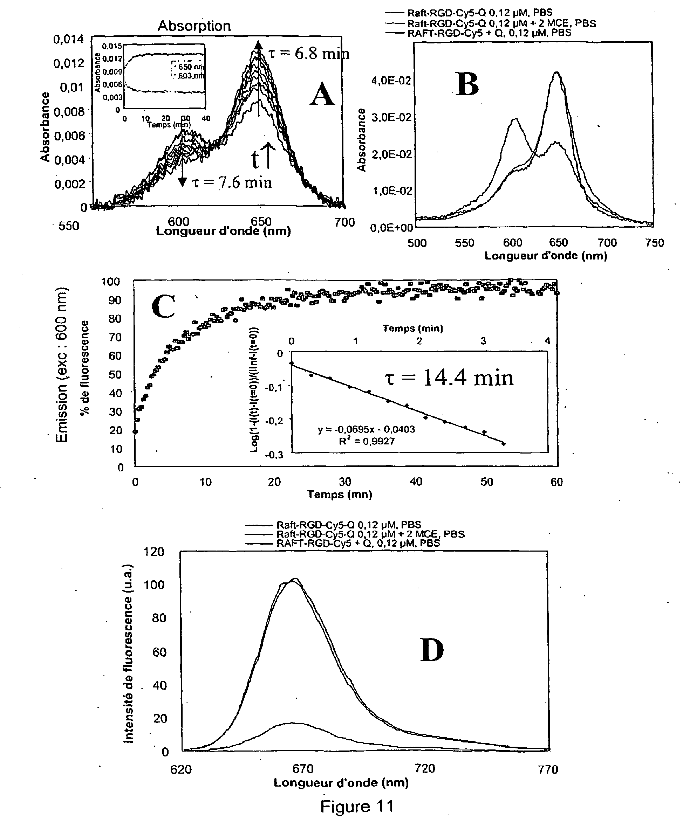

**Figure 11** **:** Absorption et fluorescence de la fonction d'imagerie Cy5-Cys-S-S-Cys-QSY21 greffée sur le RAFT-(cRGD)₄ avant et après clivage chimique du pont disulfure par le 2-mercaptoéthanol (2-MCE). **A.** Spectre d'absorption et son évolution temporelle lors de l'ajout de 2-MCE. **B.** Mise en évidence que l'ajout de 2-MCE conduit au recouvrement total du spectre d'absorption de Cy5. C. Evolution temporelle de la fluorescence de la fonction d'imagerie lors de l'ajout de 2-MCE. D. Mise en évidence que l'ajout de 2-MCE conduit au recouvrement total de la fluorescence de Cy5. (fonction d'imagerie à 0.10 µM dans du PBS 10 mM pH 7.2, [2-MCE] = 85 mM).
**Figure 12** **:** Résultats de l'injection *in vivo* des sondes moléculaires RAFT-(cRGD)₄-Cy5-Cys-S-S-Cys-Cy5, RAFT-(cRGD)₄-Cy5-Cys-S-S-Cys-QSY21, et RAFT-(cRGD)₄-Cy5-Cys-S-S-Cys (témoin) dans des souris nude, imagées par un dispositif d'imagerie de fluorescence (excitation 633 nm ; détection λ> 670 nm). Injection intraveineuse de 10 nmol de sonde/souris, dans 100 µL de PBS (pH 7.1, 9.5 mM) dans la queue des souris anesthésiées portant sur le dos une tumeur sous-cutanée de type IGROV-1 (10 10⁶ cellules injectées sous-cutané 2 semaines avant imagerie). A. Intensité de fluorescence mesurée dans la tumeur au cours du temps. B. Rapport des intensités de fluorescence mesurées dans la tumeur et dans la peau au cours du temps. C. Images obtenues 10 minutes et 5 h après injection pour les différentes sondes moléculaires.

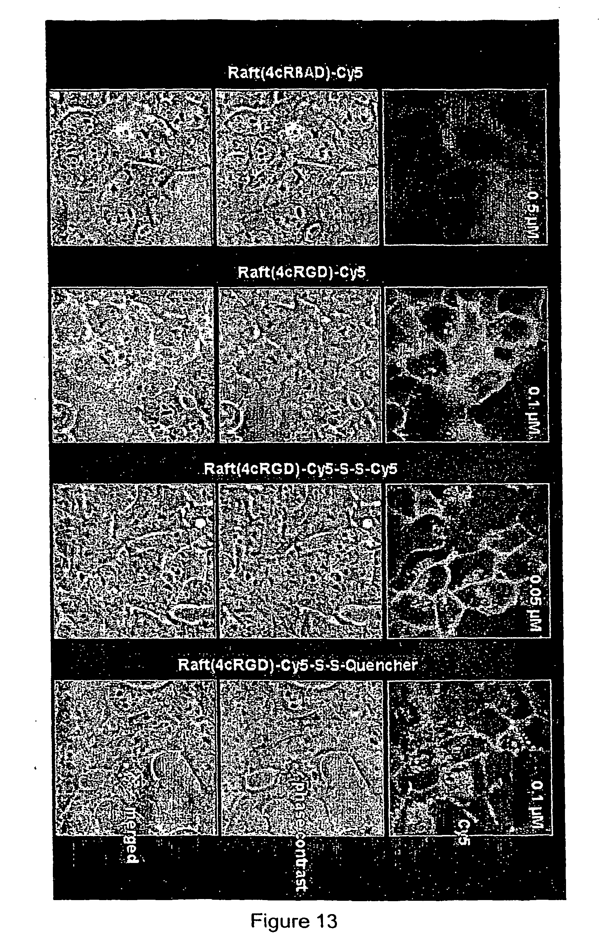

**Figure 13** **:** Pénétration des marqueurs dans les cellules, imagée par microscopie confocale à balayage laser. Cellules Hekβ3 après 1 à 2 heures d'incubation en présence d'un témoin négatif RAF(4cRAD)-Cy5 (1^{ère} colonne), en présence de RAFT (4cRGD) -Cy5 (colonne 2), RAFT (4cRGD) -Cy5-Cys-S-S-Cys-Cy5 (colonne 3), ou RAFT(4cRGD)-Cy5-Cys-S-S-Cys-Inhibiteur (colonne 4).

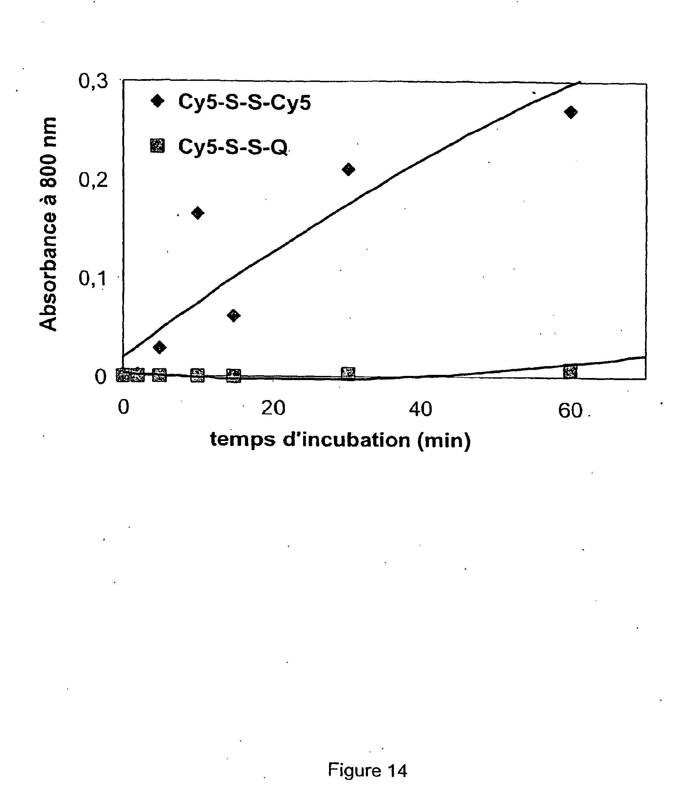

**Figure 14** **:** Toxicité comparée des fonctions d'imagerie Cy5-Cys-S-S-Cys-Cy5 et Cy5-Cys-S-S-Cys-Q. 10 mL de solution de Cy5-Cys-S-S-Cys-Cy5 ou de Cy5-Cys-S-S-Cys-Q (non greffés sur un vecteur), à environ 0.5 µM dans du PBS, sont incubés en présence de cellules TSA (environ 20.10⁶ cellules/flasque). La solution est prélevée à différents intervalles de temps.

### EXEMPLES

### Exemple 1 Un mode de réalisation de l'invention

Le mode de réalisation de l'invention décrit ci-dessous repose sur l'utilisation :
- du vecteur moléculaire RAFT décrit précédemment (Boturyn, Coll et al. 2004; Dumy, Favrot et al. 2004). L'utilisation du vecteur moléculaire RAFT apporte à l'invention des avantages particuliers qui sont les suivants :
   - sa structure moléculaire est définie précisément et bien contrôlée, contrairement à une sonde polymérique pour laquelle seul le poids moléculaire moyen est déterminé.
   - la sonde moléculaire obtenue peut être aisément purifiée selon les techniques connues de l'homme du métier (CLHP par exemple), contrairement à une sonde polymérique.
   - cette sonde moléculaire est de plus petite taille que d'autres nanoparticules, ce qui favorise son internalisation dans les cellules.
- du ligand biologique cRGD décrit précédemment. Le cyclopeptide cRGD a été choisi comme ligand biologique car il est connu depuis longtemps pour cibler les récepteurs des intégrines αᵥβ₃ surexprimés à la surface des cellules endothéliales des vaisseaux sanguins en développement (Brooks, Clark et al. 1994). Ces récepteurs sont notamment surexprimés dans différents modèles de cellules cancéreuses.
- des fonctions d'imagerie Cy5-Cys-S-S-Cys-Cy5 et Cy5-Cys-S-S-Cys-QSY21 décrites ci-dessous. Ces fonctions d'imagerie utilisent le pont disulfure entre deux cystéines (clivage catalysé enzymatiquement par les thiorédoxines dans les lysosomes et endosomes des cellules (Arunachalam, Phan et al. 2000)), le fluorophore Cy5 commercialisé par *Amersham* et l'inhibiteur de fluorescence QSY21 commercialisé par *Molecular Probes*.

### Fonction d'imagerie Cystéine (Cy5)-S-S-Cystéine (Cy5)

La structure chimique de cette molécule est représentée Figure 4. Elle a été préparée à partir de la cystéine (0.4 mg, 1.6 µmol) et de l'ester activé N-hydroxysuccinimide de la cyanine 5 (2.8 mg, 3.5 µmol) dans une solution de DMF/H₂O (6:1) à pH 8.0. Le produit est obtenu après une purification par CLHP sous forme de poudre bleue (1.2 mg, 0.8 µmol, 50 %). La Figure 5 montre que la fluorescence des fluorophores Cy5 est bien auto-inhibée initialement, et est complètement libérée par clivage chimique du pont disulfure S-S.

### Fonction d'imagerie Cystéine (Cy5)-S-S-Cystéine (QSY21)

La structure chimique de cette molécule est représentée Figure 4. Elle a été préparée à partir de Boc-cystéine(Npys) (210 mg, 0.56 mmol). Le groupe Boc est tout d'abord éliminé dans une solution de TFA /DCM (1 :1) (138 mg, 0.5 mmol, 90 %). Puis à une solution de Cystéine (*Npy*s) (1.7 mg, 6.3 µmol) on ajoute le N-hydroxysuccinimide de la Cyanine 5 (5 mg, 6.3 µmol) dans une solution DMF/H₂O (9:1) à pH 8.0. Après purification du composé, la CystéineOMe (6.1 mg, 6.1 µmol) est ajoutée et le pH ajusté à 7.0 dans l'eau. Une purification par CLHP donne le composé Cy5-Cys-SS-CysOMe sous forme de poudre bleue (2.4 mg, 2.5 µmol, 52 %). Ce composé (2.4 mg, 2.5 µmol) est alors repris avec l'ester succinimide de QSY^{®}21 (2 mg, 2.5 µmol) dans le DMF à pH 8.0. Une purification par CLHP donne le produit désiré sous forme de poudre bleue (2.2 mg, 1.3 µmol, 54 %). La Figure 6 montre que la fluorescence du fluorophore Cy5 est bien inhibée initialement par le QSY21, et est complètement libérée par clivage chimique du pont disulfure S-S.

### Validation biologique des fonctions d'imagerie

La Figure 7 montre que la fluorescence des fonctions d'imagerie n'est pas libérée lors de leur circulation dans le sang des souris. Elle montre aussi qu'aucune adsorption non spécifique de la fonction d'imagerie Cy5-Cys-S-S-Cys-QSY21 n'a lieu sur les protéines sanguines, alors que 25 % d'absorption se produit pour la fonction Cy5-Cys-S-S-Cys-Cy5.

La Figure 8 confirme ces résultats : après injection par voie intraveineuse des fonctions d'imagerie, aucune fluorescence n'est observée, contrairement au cas du fluorophore Cy5 non fonctionnalisé.

### Synthèse de la sonde moléculaire cystéine (Cy5)-S-S-cystéine(Cy5)RAFTc[RGDfK-]₄

La Figure 9 donne la structure de la molécule cystéine(Cy5)-S-S-cystéine(Cy5) RAFTc[RGDfK-]₄. Le peptide Cystéine(Npys)RAFTc[-RGDfK]₉ (10 mg, 2.26 µmol) est repris dans 0.5 mL de DMF/PBS pH 4.8 3/1 avec la Cystéine (0.3 mg, 2.7 µmol). Le produit est purifié par CLHP (5.3 mg, 53 %). Le peptide Cystéine-S-S-CystéineRAFTc[-RGDfK]₄ (3.5 mg, 0.79 µmol) est repris dans 300 µL de DMF avec le N-hydroxysuccinimide de la Cyanine 5 (1.9 mg, 2.4 µmol). Le produit final est purifié par CLHP et obtenu sous forme de poudre bleue (2.4 mg, 54 %).

La Figure 10 montre que la fluorescence des fluorophores Cy5 est bien auto-inhibée initialement, et est complètement libérée par clivage chimique du pont disulfure S-S.

### Synthèse de la sonde moléculaire cystéine(Cy5)-S-S-cystéine(QSY21)RAFTc[RGDfK-]₄

La Figure 9 donne la structure de la molécule cystéine(Cy5)-S-S-cystéine(QSY21)RAFTc[RGDfK-]₄. Le peptide Cystéine-S-S-BocCystéineRAFTc[RGDfK-]₄ (19.9 mg, 4.45 µmol) est repris dans 0.4 mL de DMF avec le N-hydroxysuccinimide de la Cyanine 5 (3.1 mg, 3.91 µmol). Le solvant est éliminé sous pression réduite puis le peptide Cystéine(Cy5)-S-S-BocCystéineRAFTc[RGDfK-]₄ est repris dans 6 mL de TFA/TIS/H₂O 95/2.5/2.5 et purifié par CLHP et obtenu sous forme de poudre bleue (11.5 mg, 51%). Le peptide Cystéine(Cy5)-S-S-CystéineRAFTc[RGDfK-]₄ est repris dans 300 µL de DMF avec le N-hydroxysuccinimide de QSY21 (1.13 mg, 1.39 µmol) et purifié par CLHP et obtenu sous forme de poudre bleue (1.2 mg, 20%).

La Figure 11 montre que la fluorescence du fluorophore Cy5 est bien auto-inhibée initialement, et est complètement libérée par clivage chimique du pont disulfure S-S.

### Exemple 2 : Imagerie optique de tumeurs dans la souris nude

Les vecteurs moléculaires RAFT-(cRGD)₄-Cy5-Cys-S-S-Cys-Cy5 et RAFT-(cRGD)₄-Cy5-Cys-S-S-Cys-QSY21 peuvent être utilisés pour imager spécifiquement les cellules endothéliales de tumeurs sur-exprimant des récepteurs aux intégrines αᵥβ₃, telles des cellules IGROV-1 (modèle de cancer ovarien humain) implantées sous-cutanées dans une souris nude.

L'imagerie optique se fait par un dispositif d'imagerie de fluorescence commercial ou prototype. Les sondes injectées par voie intraveineuse restent non fluorescentes dans le corps de l'animal hors de la tumeur (Figure 8). Après ciblage spécifique des tumeurs par le ligand RGD, les sondes sont internalisées dans les cellules tumorales. En milieu intracellulaire, les fonctions d'imagerie sont activées (Figure 12). L'objet de l'invention permet donc l'imagerie optique non invasive de tumeurs par un dispositif d'imagerie de fluorescence *in vivo*. Il permet par conséquent de suivre l'évolution de tumeurs au cours du temps ou en réponse à un traitement thérapeutique.

### Modèles biologiques et injection des marqueurs

Les souris utilisées sont des souris *nude* femelles de 6 à 8 semaines, et maintenues sous conditions sans pathogènes. Les cellules IGROV-1 (modèle de cancer ovarien humain) sont cultivées dans un milieu de culture RPMI 1640 comportant 1% de glutamine, 10% de FCS, 50 U/mL des pénicilline, 50 µg/mL de streptomycine. Les cellules sont maintenues à 37°C sous atmosphère humide avec 5% de CO₂. 10 10⁶ cellules sont injectées en sous-cutané dans le dos des souris 2 semaines avant injection des vecteurs moléculaires RAFT-(cRGD)₄-Cy5-Cys-S-S-Cys-Cy5 et RAFT-(cRGD)₄-Cy5-Cys-S-S-Cys-QSY21. Ceux-ci sont injectés dans la queue par voie intraveineuse dans 100 µL de PBS (pH 7.1, 9.5 mM), à des doses de 10 nmol/ souris.

### Dispositif d'imagerie de fluorescence par réflectance

Les souris anesthésiées sont imagées avec un dispositif d'imagerie de fluorescence par réflectance (FRI = *Fluorescence Reflectance Imaging*), comportant comme source d'excitation une couronne de LEDs munies de filtres interférentiels, émettant à 633 nm (puissance d'éclairement 50 µW.cm⁻²). Les images sont recueillies après filtration par un filtre coloré RG665 de densité optique >5 à la longueur d'onde d'excitation par une caméra CCD (Orca BTL, Hamamatsu) avec un temps d'exposition de 100 ms. Les signaux sont quantifiés à l'aide d'un logiciel de traitement d'images.

### Résultats obtenus

Les résultats obtenus sont représentés sur la Figure 12. Il apparaît que les sondes activables RAFT-(cRGD)₄-Cy5-Cys-S-S-Cys-Cy5 et RAFT-(cRGD)₄-Cy5-Cys-S-S-Cys-QSY21 conduisent à plus de signal dans la tumeur que la sonde RAFT-(cRGD)₄-Cy5-Cys-S-S-cystéine, et conduisent à une apparition progressive du signal dans la tumeur, au fur et à mesure de l'activation de la sonde (puis à son déclin par élimination), alors que la sonde RAFT-(cRGD)₄-Cy5-Cys-S-S-cystéine conduit à un signal qui décroît très rapidement au cours du temps (Figure 12A). En conséquence, le contraste, c'est à dire le rapport de signal obtenu entre la tumeur et la peau augmente en passant de la sonde RAFT-(cRGD)₄-C-y5-Cys-S-S-cystéine aux sondes RAFT-(cRGD)₄-Cy5-Cys-S-S-Cys-Cy5 et RAFT-(cRGD)₄-Cy5-Cys-S-S-Cys-QSY21 (Figure 12B).

Les fonctions d'imagerie décrites ici permettent donc bien d'obtenir, par le fait que la fluorescence de la sonde soit activée progressivement et de façon ciblée dans les tumeurs, un bruit de fond plus faible dans le reste du corps de l'animal que la sonde classique ciblée correspondante.

### Exemple 3 : Comparaison des fonctions d'imagerie Cy5-Cys-S-S-Cys-Cy5 et Cy5-Cys-S-S-Cys-Q

La pénétration dans les cellule, des sondes RAFT-(cRGD)₄-Cy5, RAFT-(cRGD)₄-Cy5-Cys-S-S-Cys-Cy5 et RAFT-(cRGD)₄-Cy5-Cys-S-S-Cys-QSY21 a été observée sur des cellules Hekβ3, après 1 à 2 heures d'incubation. Les résultats, présentés à la figure 13, montrent qu'avec la sonde RAFT-(cRGD)₄-Cy5, la fluorescence reste à la périphérie des cellules (colonne 2) ; les cellules incubées avec RAFT-(cRGD)₄-Cy5-Cys-S-S-Cys-QSY21 présentent un fluorescence nette à l'intérieur des cellules (colonne 4), tandis qu'avec la sonde RAFT-(cRGD)₄-Cy5-Cys-S-S-Cys-Cy5 (colonne 3), une grande quantité de fluorescence est encore visible au niveau de la membrane des cellules, ce qui prouve que la sonde ne rentre pas bien. Ces résultats illustrent le fait qu'une molécule neutre (en l'occurrence, RAFT-(cRGD)₄-Cy5-Cys-S-S-Cys-QSY21) pénètre mieux dans les cellules qu'une molécule chargée.

La toxicité des fonctions d'imagerie Cy5-Cys-S-S-Cys-Cy5 et Cy5-Cys-S-S-Cys-Q a été comparée. 10 mL de solution de Cy5-Cys-S-S-Cys-Cy5 ou de Cy5-Cys-S-S-Cys-Q (non greffés sur un vecteur), à environ 0.5 µM dans du PBS, ont été incubés en présence de cellules TSA (environ 20.10⁶ cellules/flasque). La solution a été prélevée à différents intervalles de temps.

Les résultats sont présentés à la figure 14. On observe que dans le cas du Cy5-Cys-S-S-Cys-Cy5, la solution se trouble, ce qui se traduit par une diffusion importante de la solution, donc une augmentation de l'absorbance, notamment à 800 nm, longueur d'onde pour laquelle les molécules Cy5, Cy5-Cys-S-S-Cys-Q et Cy5-Cys-S-S-Cys-Cy5 n'absorbent normalement pas. Ce trouble de la solution, qui la rend diffusante, est interprété par le fait que les cellules se sont décollées des parois de la flasque de culture, indiquant leur destruction par Cy5-Cys-S-S-Cys-Cy5. Dans le cas de l'incubation en présence de Cy5-Cys-S-S-Cys-Q, la mort cellulaire (et donc le décollement des cellules) ne commence à exister qu'après 1 h d'incubation et est bien moindre. La molécule Cy5-Cys-S-S-Cys-Cy5 à 0.5 µM entraîne donc la mort cellulaire des cellules TSA, et ce dès les premières minutes d'incubation, contrairement à la molécule Cy5-Cys-S-S-Cys-Q.

### REFERENCES

Arunachalam, B., U. Phan, et al. (2000). "Enzymatic reduction of disulfide bonds in lysosomes: characterization of a gamma-interferon-inducible lysosomal thiol reductase (GILT)." Proceedings of the National Academy of Sciences, USA 97(2): 745-750.
Boturyn, D., J. L. Coll, et al. (2004). "Template assembled cyclopeptides as multimeric system for integrin targeting and endocytosis." J. Am. Chem. Soc. 126(18): 5730-5739.
Brooks, P. C., R. A. Clark, et al. (1994). "Requirement of vascular integrin alpha v beta 3 for angiogenesis." Science 264: 569-571.
Curnis, F., G. Arrigoni, et al. (2002). "Differential binding of drugs containing the NGR motif to CD13 isoforms in tumor vessels, epithelia, and myeloid cells." Cancer Res 62(3): 867-74.
Dumy, P., M. Favrot, et al. (2004). WO2004/026894, "Synthesis and characterization of novel systems for guidance and vectorization of molecules of thera^peutic interst towards target cells'.
Lakowicz, J. R. (1999). Principles of Fluorescence Spectroscopy, 2nd edition. New York, Kluwer Academics/Plenum Publishers.
Josephson,L. (2002). "Near-Infrared Fluorescent Nanoparticles as Combined Mr/Optical Imaging Probes" Bioconjugate. Chem. 13(3) : 554-560.

## Revendications

1. Vecteur biologique, comprenant un cyclodécapeptide RAFT auquel sont liées
(i) une molécule assurant le ciblage du vecteur, et
(ii) une fonction d'imagerie activable assurée par un fluorophore F relié à un inhibiteur de fluorescence par un bras clivable en milieu intracellulaire, ledit bras comportant un pont disulfure,
ledit vecteur étant destiné à être internalisé dans les cellules cibles.

2. Vecteur selon la revendication 1, **caractérisé en ce que** le bras clivable en milieu intracellulaire est constitué de deux cystéines reliées par un pont disulfure.

3. Vecteur selon l'une quelconque des revendications 1 à 2, **caractérisé en ce que** le ciblage est assuré par au moins un ligand biologique reconnu par un récepteur surexprimé à la surface de certaines cellules.

4. Vecteur selon la revendication 3, **caractérisé en ce que** le ligand biologique est choisi dans le groupe constitué par un peptide, une protéine, un anticorps, un sucre, un oligonucléotide, une molécule organique, et un complexe organométallique.

5. Vecteur selon la revendication 3 ou la revendication 4, **caractérisé en ce que** le ligand biologique est un peptide comportant le motif RGD, ou le motif peptidique NGR.

6. Vecteur selon l'une quelconque des revendications 1 à 5, **caractérisé en ce qu'**une drogue est en outre liée audit vecteur.

7. Vecteur selon la revendication 6, **caractérisé en ce que** la drogue est clivée du vecteur lors de l'activation de la fonction d'imagerie dans le milieu intracellulaire.

8. Vecteur selon la revendication 7, **caractérisé en ce qu'**après clivage dudit bras clivable, l'inhibiteur reste lié au vecteur et le fluorophore reste lié à la drogue.

9. Vecteur selon la revendication 7, **caractérisé en ce qu'**après clivage dudit bras clivable, le fluorophore reste lié au vecteur et l'inhibiteur reste lié à la drogue.

10. Vecteur selon l'une quelconque des revendications 1 à 9, **caractérisé en ce que** l'inhibiteur de fluorescence est un deuxième fluorophore pouvant absorber le rayonnement émis par F et le réémettre à une autre longueur d'onde.

11. Vecteur selon l'une quelconque des revendications 1 à 9, **caractérisé en ce que** le fluorophore F est un fluorophore s'auto-inhibant et l'inhibiteur de fluorescence est le même fluorophore F.

12. Vecteur selon l'une quelconque des revendications 1 à 9, **caractérisé en ce que** l'inhibiteur de fluorescence est un inhibiteur non fluorescent.

13. Vecteur selon l'une quelconque des revendications 1 à 12, **caractérisé en ce qu'**il est électriquement neutre avant activation de la fonction imagerie.

14. Vecteur selon la revendication 13, **caractérisé en ce que** l'activation de la fonction imagerie crée deux entités moléculaires de charges opposées.

15. Vecteur selon l'une quelconque des revendications 1 à 9 et 12 à 14, **caractérisé en ce que** la fonction d'imagerie est assurée par le fluorophore Cy5, relié par un pont disulfure à un inhibiteur de sa fluorescence.

16. Utilisation d'un vecteur selon la revendication 8 ou la revendication 10, pour la fabrication d'une composition destinée à imager la délivrance, puis la biodistribution d'une drogue.

17. Utilisation d'un vecteur selon la revendication 10, pour la fabrication d'une composition destinée à imager simultanément la biodistribution dudit vecteur et la délivrance et la biodistribution d'une drogue initialement liée audit vecteur.

18. Utilisation d'un vecteur biologique selon l'une quelconque des revendications 1 à 15, pour la fabrication d'une composition destinée à imager spécifiquement une zone tissulaire.

## Claims

1. Biological vector comprising a RAFT cyclodecapeptide to which there are bonded
(i) a molecule providing targeting of the vector, and
(ii) an activatable imaging function provided by a fluorophore F which is connected to a fluorescence inhibitor by an arm which is cleavable in intracellular medium, said arm comprising a disulfide bridge,
said vector being intended to be internalised in the target cells.

2. Vector according to claim 1, **characterised in that** the arm which is cleavable in intracellular medium is constituted by two cysteines connected by a disulfide bridge.

3. Vector according to either claim 1 or claim 2, **characterised in that** targeting is provided by at least one biological ligand recognised by a receptor overexpressed at the surface of certain cells.

4. Vector according to claim 3, **characterised in that** the biological ligand is selected from the group consisting of a peptide, a protein, an antibody, a sugar, an oligonucleotide, an organic molecule and an organometallic complex.

5. Vector according to claim 3 or claim 4, **characterised in that** the biological ligand is a peptide comprising the RGD motif or the NGR peptide motif.

6. Vector according to any one of claims 1 to 5, **characterised in that** a drug is further bonded to said vector.

7. Vector according to claim 6, **characterised in that** the drug is cleaved from the vector on activation of the imaging function in the intracellular medium.

8. Vector according to claim 7, **characterised in that**, after cleavage of said cleavable arm, the inhibitor remains bonded to the vector and the fluorophore remains bonded to the drug.

9. Vector according to claim 7, **characterised in that**, after cleavage of said cleavable arm, the fluorophore remains bonded to the vector and the inhibitor remains bonded to the drug.

10. Vector according to any one of claims 1 to 9, **characterised in that** the fluorescence inhibitor is a second fluorophore which is able to absorb the radiation emitted by F and re-emit it at a different wavelength.

11. Vector according to any one of claims 1 to 9, **characterised in that** the fluorophore F is a self-inhibiting fluorophore and the fluorescence inhibitor is the same fluorophore F.

12. Vector according to any one of claims 1 to 9, **characterised in that** the fluorescence inhibitor is a non-fluorescent inhibitor.

13. Vector according to any one of claims 1 to 12, **characterised in that** it is electrically neutral prior to activation of the imaging function.

14. Vector according to claim 13, **characterised in that** activation of the imaging function creates two molecular entities of opposite charges.

15. Vector according to any one of claims 1 to 9 and 12 to 14, **characterised in that** the imaging function is provided by the Cy5 fluorophore, which is connected by a disulfide bridge to an inhibitor of its fluorescence.

16. Use of a vector according to claim 8 or claim 10 in the production of a composition for imaging the delivery and then the biodistribution of a drug.

17. Use of a vector according to claim 10 in the production of a composition for simultaneously imaging the biodistribution of said vector and the delivery and biodistribution of a drug initially bonded to said vector.

18. Use of a biological vector according to any one of claims 1 to 15 in the production of a composition for specifically imaging a tissue area.

## Patentansprüche

1. Biologischer Vektor, umfassend ein Cyclodecapeptid RAFT, an das
(i) ein Molekül, das das Targeting des Vektors gewährleistet, und
(ii) eine aktivierbare Bildgebungsfunktion, die durch einen Fluorophor F gewährleistet wird, welcher an einen Fluoreszenzinhibitor über einen in intrazellulärem Milieu abspaltbaren Arm, wobei der Arm eine Disulfidbrücke umfasst, gebunden ist,
gebunden sind,
wobei der Vektor dazu bestimmt ist, in die Zielzellen internalisiert zu werden.

2. Vektor gemäß Anspruch 1, **dadurch gekennzeichnet, dass** der im intrazellulären Milieu abspaltbare Arm aus zwei Cysteinen besteht, die durch eine Disulfidbrücke verbunden sind.

3. Vektor gemäß einem der Ansprüche 1 bis 2, **dadurch gekennzeichnet, dass** das Targeting durch wenigstens einen biologischen Liganden sichergestellt wird, der durch einen Rezeptor erkannt wird, der an der Oberfläche bestimmter Zellen überexprimiert wird.

4. Vektor gemäß Anspruch 3, **dadurch gekennzeichnet, dass** der biologische Ligand aus der Gruppe, bestehend aus einem Peptid, einem Protein, einem Antikörper, einem Zucker, einem Oligonucleotid, einem organischen Molekül und einem metallorganischen Komplex, ausgewählt ist.

5. Vektor gemäß Anspruch 3 oder Anspruch 4, **dadurch gekennzeichnet, dass** der biologische Ligand ein Peptid ist, das das Motiv RGD oder das Peptidmotiv NGR umfasst.

6. Vektor gemäß einem der Ansprüche 1 bis 5, **dadurch gekennzeichnet, dass** außerdem ein Wirkstoff bzw. eine Droge an den Vektor gebunden ist.

7. Vektor gemäß Anspruch 6, **dadurch gekennzeichnet, dass** der Wirkstoff bzw. die Droge während der Aktivierung der Bildgebungsfunktion im intrazellulären Milieu vom Vektor abgespalten wird.

8. Vektor gemäß Anspruch 7, **dadurch gekennzeichnet, dass** der Inhibitor nach Abspaltung des abspaltbaren Arms am Vektor gebunden bleibt und der Fluorophor an dem Wirkstoff bzw. der Droge gebunden bleibt.

9. Vektor gemäß Anspruch 7, **dadurch gekennzeichnet, dass** der Fluorophor nach Abspaltung des abspaltbaren Arms am Vektor gebunden bleibt und der Inhibitor am Wirkstoff bzw. der Droge gebunden bleibt.

10. Vektor gemäß einem der Ansprüche 1 bis 9, **dadurch gekennzeichnet, dass** der Fluoreszenzinhibitor ein zweiter Fluorophor ist, der die durch F emittierte Strahlung absorbieren kann und sie mit einer anderen Wellenlänge wieder emittieren kann.

11. Vektor gemäß einem der Ansprüche 1 bis 9, **dadurch gekennzeichnet, dass** der Fluorophor F ein sich selbst inhibierender Fluorophor ist und der Fluoreszenzinhibitor der selbe Fluorophor F ist.

12. Vektor gemäß einem der Ansprüche 1 bis 9, **dadurch gekennzeichnet, dass** der Fluoreszenzinhibitor ein nicht-fluoreszierender Inhibitor ist.

13. Vektor gemäß einem der Ansprüche 1 bis 12, **dadurch gekennzeichnet, dass** er vor Aktivierung der Bildgebungsfunktion elektrisch neutral ist.

14. Vektor gemäß Anspruch 13, **dadurch gekennzeichnet, dass** die Aktivierung der Bildgebungsfunktion zwei Moleküleinheiten mit entgegengesetzten Ladungen erzeugt.

15. Vektor gemäß einem der Ansprüche 1 bis 9 und 12 bis 14, **dadurch gekennzeichnet, dass** die Bildgebungsfunktion durch den Fluorophor Cy5 gewährleistet ist, der durch eine Disulfidbrücke an einen Inhibitor seiner Fluoreszenz gebunden ist.

16. Verwendung eines Vektors gemäß Anspruch 8 oder gemäß Anspruch 10 für die Herstellung einer Zusammensetzung, die dazu bestimmt ist, die Abgabe, danach die Bioverteilung eines Wirkstoffs bzw. einer Droge bildlich darzustellen.

17. Verwendung eines Vektors gemäß Anspruch 10 für die Herstellung einer Zusammensetzung, die dazu bestimmt ist, gleichzeitig die Bioverteilung des Vektors und die Abgabe und die Bioverteilung eines Wirkstoffs bzw. einer Droge, der/die anfangs an den Vektor gebunden war, bildlich darzustellen.

18. Verwendung eines biologischen Vektors gemäß einem der Ansprüche 1 bis 15 für die Herstellung einer Zusammensetzung, die dazu bestimmt ist, einen Gewebebereich spezifisch bildlich darzustellen.
